# EUROPEAN PATENT SPECIFICATION

(11) **EP 2 490 675 B1**
(45) Date of publication and mention of the grant of the patent: **23.01.2019**
(21) Application number: 09741235.7
(22) Date of filing: 22.10.2009
(51) Int. Cl.: A61K 9/20, A61K 9/28, A61K 31/785

(54) **PHARMACEUTICAL COMPOSITIONS OF SEVELAMER**
PHARMAZEUTISCHE ZUSAMMENSETZUNG ENTHALTEND SEVELAMER
COMPOSITION PHARMACEUTIQUE COMPRENANT SEVELAMER

(43) Date of publication of application: 29.08.2012
(73) Proprietor: Synthon B.V., 6545 CM Nijmegen (NL)
(72) Inventor: OSINGA, Niels, Jaap, NL-6524 JH Nijmegen (NL)
(74) Representative: Mendivil Gil, Maria Dolores
(86) International application number: PCT/EP2009/007586
(87) International publication number: WO 2011/047700

(56) References cited:
- EP-A1- 0 997 148
- WO-A1-2007/094779
- WO-A1-2009/034540
- WO-A1-2009/156014
- WO-A2-01/28527
- DE-A1-102005 024 614

## Description

The present invention relates to pharmaceutical compositions for oral administration comprising sevelamer carbonate, wherein the composition is free of crystalline cellulose, and (low-substituted) hydroxypropyl cellulose or other binding agents.

### Background of the Invention

Sevelamer is a non-absorbed phosphate binding polymer used in the treatment for the control of serum phosphorus in patients with Chronic Kidney Disease (CKD). Its chemical structure is as follows :

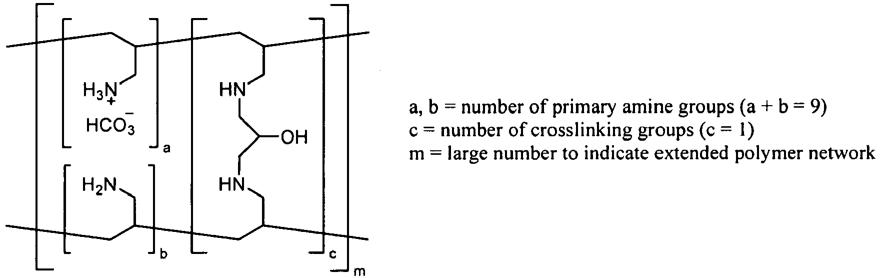

The compound contains multiple amines that become partially protonated in the intestine and interact with phosphate ions through ionic and hydrogen bonding. By binding phosphate in the gastrointestinal tract facilitating phosphorus excretion in feces, Sevelamer lowers the plasma phosphorus concentration. The use of Sevelamer and its pharmaceutical compositions, and processes for its preparation are disclosed in EP 0716606, EP 0831857, EP 1133989 and EP 1676581.

Sevelamer may form acid addition salts. In existing medicinal products, Sevelamer is marketed, i.a., as Sevelamer carbonate (Renvela®). Renvela® contains microcrystalline cellulose, sodium chloride and zinc stearate as inactive ingredients. The tablets are coated with hypromellose and diacetylated monoglycerides.

According to the approved product information , sevelamer carbonate needs to be taken with meals. Therefore, rapid disintegration of a sevelamer carbonate containing tablet is conditional for phosphate binding efficacy, as it brings sevelamer in contact with food and allows binding of phosphate before it is absorbed by the intestinal tissue. Furthermore, the therapeutic dose is very high; the marketed tablets of sevelamer carbonate comprise 800 mg of the active substance per tablet and the daily dose may be up to 14 g per day.

Sevelamer carbonate is a compound of hygroscopic nature, wherein the adsorption of water is associated with serious changes in the volume. Sevelamer carbonate tablet cores swell if uptake of moisture occurs. This can lead to rupture of the coating around the core, if the coating does not possess flexibility. Flexibility of the coating is particularly important for 'in-use' stability as patients might not always store the tablets according to the label recommendations.

Tablets comprising sevelamer, particularly sevelamer hydrochloride and sevelamer carbonate are known in the art.

WO 98/44933 and WO 00/22008 both describe tablets containing sevelamer, having an average particle size of 400 microns or less and 90% of the particles are less than 500 microns, together with crystalline cellulose (binder/diluent) and/or low-substituted hydroxypropyl cellulose (binder). The examples disclose 200 mg sevelamer compositions comprising 32.5% to 50.0% microcrystalline cellulose.

The applications further disclose that crystalline cellulose and low- substituted hydroxypropyl cellulose are desired to obtain a tablet disintegration time of less than 15 minutes, which is not obtained by using additives other than low- substituted hydroxypropyl cellulose or crystalline cellulose. The inventors describe in Example 1 (Table 1) the use of lactose and mannitol; however, no sufficient hardness (at least 6 KP) was reached.

The applications further describe a film-coated tablet wherein the film-coating may be made of water-soluble film bases such as hydroxypropylmethylcellulose and acrylic copolymers.

WO 01/28527 describes a tablet comprising a core and a coating wherein at least 95% by weight of the core comprises sevelamer. Further, the application describes water-based coatings for sevelamer tablets. The examples disclose the use of hydroxypropylmethylcellulose (HPMC) only.

WO 2006/050315 describes lactose amongst others as a suitable pharmaceutical carrier for sevelamer formulations, but provides no further information on the applicability.

WO 2008/062437 describes the use of mannitol as a preferred diluent in the range of 5% to 21% in combination with a binder added by a wet granulation process. Example 7 discloses the use of 10.8% mannitol in combination with ethyl cellulose (binder). The application describes that tablet compositions which use lactose as a diluent show discoloration as the tablets turn to yellowish brown color due to Maillard reaction.

Further, the application mentions several polymers for film coating. The coating may be used in the range of 3.0% to 8.0% by weight of total composition. The examples describe the use of a film coating aqueous process until a weight gain in the range of 4.0% to 6.0% is achieved (no specific film-coating polymer mentioned).

WO 2009/034540 describes compositions comprising sevelamer in an amount less than 80% by weight of composition. Furthermore, the application describes compositions comprising hydroxypropyl cellulose (HPC; binder), free of crystalline cellulose and low-substituted hydroxypropyl cellulose.

The examples disclose 400 mg sevelamer HCl compositions comprising 37.1% to 38.0% of diluent, lactose (Examples 1 and 2), mannitol (Example 10) and starch (Example 11). The examples disclose also 800 mg sevelamer compositions comprising 22% to 23.5% of lactose in combination with binder (Examples 3, 4, 5, 6, 13 and 14). Compositions comprising 15.5% to 16.5% mannitol in combination with binder are also disclosed (Examples 7, 8, and 9).

The application further mentions the use of film-forming polymers including Eudragit® or Opadry®, but provides no further information on applicability of such coating.

In all known 800 mg sevelamer carbonate formulations, binders are used, optionally in combination with a diluent, for developing a suitable sevelamer composition with an acceptable disintegration time. Although WO 2009/034540 disclosed the use of a diluent only, the amount of diluent used in the examples exceeds, however, the acceptable properties for an 800 mg sevelamer tablet.

Due to the high dose intake of sevelamer carbonate (800 mg per tablet), there is a need to develop an alternative composition of sevelamer carbonate with with low amounts of excipients and an acceptable disintegration time, which is easy to produce using direct compression manufacturing techniques.

Additionally, there is a need to develop an alternative composition of sevelamer carbonate with an acceptable disintegration time using a suitable flexible film-coating, wherein the coating is able to allow moisture uptake during shelf-life storage time and does not substantially increase the disintegration time of the sevelamer carbonate comprising tablets.

### Summary of the Invention

The present invention provides a pharmaceutical immediate release tablet comprising a core comprising 70-85 weight per cent of sevelamer carbonate, calculated as an anhydrous compound, 10-25 weight percent of lactose monohydrate and, optionally , a water soluble film coat surrounding the core.

Advantageously, the tablet comprises 800 mg of sevelamer carbonate.

Advantageously, the total mass of the tablet, inclusive the optional coating, does not exceed 1150 mg.

Advantageously, the film coat comprises polyvinyl alcohol-polyethylene glycol graft copolymer.

Advantageously, the amount of the film coat is 2-5 % of the weight of the tablet core.

Advantageously, the tablet does not comprise an added monovalent anion source and/or a disintegrant and/or a surfactant.

Advantageously the tablet has a hardness of about 150 N and higher and/or has a disintegration of less than 10 minutes using a conventional tablet disintegration apparatus (Pharmatest) in accordance with the European Pharmacopoeia (Ph.Eur 2.9.1) in purified water without using the disk.

In a second aspect, the invention provides a process for making immediate release tablets comprising sevelamer carbonate , said process comprising tabletting, by a direct compression, a dry mixture of 70-85 weight per cent of sevelamer carbonate, calculated as an anhydrous compound, 10-25 weight percent of monohydrate of lactose and ,optionally ,other excipients.

Optionally, the tabletting step is followed by a step of coating the formed tablets by a water soluble film coat; advantageously the coat comprises polyvinyl alcohol-polyethylene glycol graft copolymer.

In further aspects, the invention relates to the use of tablets according to the invention as medicaments for the control and/or adjustment of the level of serum phosphorus, and to the use of polyvinyl alcohol-polyethylene glycol graft copolymer for making coated tablets comprising sevelamer, particularly sevelamer carbonate.

### Detailed description of the invention

The objective of the invention is to provide directly compressible film-coated tablets comprising sevelamer carbonate with short disintegration time, high drug load, and sufficient hardness. Furthermore, another object of the invention was to provide a suitable film-coating material protecting the hygroscopic tablet core comprising sevelamer carbonate but not affecting the overall release of the drug.

Due to hygroscopic nature of sevelamer carbonate associated with serious volume gain during the uptake of water, the useful tabletting procedures are limited to direct compression, dry granulation and non-aqueous wet granulation. The non-aqueous wet granulation (a granulation using organic solvents, e.g. alcohols) is laborious, not applicable in many pharmaceutical plants, more expensive, and environment-unfriendly. Therefore, the aim of the research was to provide a composition of the tablet core that was tablettable by a dry process, preferably by the direct compression and most preferably by a direct compression without any wetting step.

Due to the therapeutical dose of sevelamer carbonate, which requires to make the final tablets comprising 800 mg of the active, the resulting tablet is generally very big. It is commonly accepted that tablets having the total weight of 1000-1100 mg are still swallowable, except, of course, for disabled patients. Therefore, the aim of the research was to provide a composition having at least 70 %, preferably between 70 and 85 weight % of the sevelamer carbonate (calculated as an anhydrous compound) in the core, in other words, a composition having, in total, only 15-30 % of other excipients. The "other excipients" also include water , which is present in the composition and originates both from the active substance (the active substance may inherently contain up to 8 % of water) and from the excipients and environment; even when a dry tabletting process is used, some water may be adsorbed into the composition from the environment. In average, the overall content of water in the composition is generally about 3 %.

The role of excipients in the immediate release tablet comprising 70-85 % of sevelamer carbonate is generally to bind the composition in the tabletting process into a sufficiently hard and stable tablet and, on the other hand, to allow sufficiently rapid disintegration of the tablet matrix in the stomach environment. It was proven that it is impossible to obtain a tablet with sufficient hardness and disintegration just by tabletting sevelamer carbonate without any binding excipients.

In the key aspect, various water soluble diluents and disintegrant excipients suitable for direct compression tabletting process were tested in respect to the final disintegration time with the goal to provide a stable hard tablet comprising from 70 to 85 % of sevelamer carbonate , preferably comprising 800 mg of the active compound , calculated as an anhydrous substance, within a tablet having the overall mass of about 1100 mg, whereby the produced tablet may have exhibit the disintegration time of less than 10 minutes, when measured according to Ph.Eur. standard disintegration test (Art. 2.9.1) in purified water without using the disk. The experimental arrangements and results of the corresponding tests are provided in Experimental Example A below.
a) In respect to the tested excipients, it was found that lactose monohydrate, mannitol and the mixtures of microcrystalline cellulose with silicified cellulose provide the tablets having the shortest disintegration times. Only with these three excipients, the required tablet disintegration times of < 10 minutes were obtained. The use of many other excipients - such as anhydrous lactose, povidone, co-povidone, HPC with low viscosity, compressible sugar, isomalt and starch-lead to substantially longer disintegration times (> 10 minutes). No explanation was found for this surprising result. It was expected that the use of any well soluble excipient and /or swelling excipient would lead to short tablet disintegration time. However, in the case of high loaded sevelamer carbonate formulation, many excipients known for high solubility and/or swelling capacity did not lead to short tablet disintegration time, except for lactose monohydrate, mannitol and cellulose/silicified cellulose mixtures. A relation between the binding strength of the excipients and the disintegration time might have been expected, but also this relation was not found.

Among these suitable excipients, the lactose monohydrate has the best disintegration properties in the sevelamer carbonate tablet compositions. It provides the sevelamer carbonate tablets with sufficient hardness and smooth surface, i.e. well suitable for the subsequent film-coating. Furthermore, mannitol, like many other polyols, is not a suitable excipient for tablets that should be used by patients with kidney diseases in high doses. Laxative effects of mannitol are more pronounced with kidney disease patients due to the limited water intake associated with dialysis, and absorbed mannitol exerts uncontrolled diuretic effect on the kidneys. Accordingly , lactose monohydrate , in an amount of 10-25 % and most preferably 20-25 % of the tablet composition, is the most suitable excipient for making a high loaded immediate release tablet comprising sevelamer carbonate. Both the spray- dried and the granulated lactose monohydrate are suitable in the invented tablets.

Contrary to certain prejudice, the presence of lactose monohydrate in the composition does not affect the stability of the phosphate binding capacity. Sevelamer is the molecule that comprises a large extent of primary amino groups. Lactose is a component that is generally known for its reactivity with primary amino groups by a Maillard-type condensation reaction. The handbooks state that lactose and primary amines are generally incompatible. The reaction of lactose with the primary amine groups of Sevelamer would reduce the phosphate binding capacity, as the intact primary amino groups are essential for the phosphate binding functionality of sevelamer. However, surprisingly, the phosphate binding capacity of the tested tablets remained stable as shown in the Example B . Furthermore, no colourisation to brown or yellow-brown colour due to a Maillard-type reaction was observed.

The above tablet composition of sevelamer carbonate with lactose monohydrate also exhibits good stability of the disintegration time. While WO 2006/050315 indicates that the disintegration time of Sevelamer carbonate tablets increases with time of storage if no monovalent anion source was added in tablets and the marketed Renvela product contains the added sodium chloride according to the label, the composition of the present invention, however, does not comprise any added monovalent anion source and still the composition exhibits a stable disintegration time.
b) Use of disintegrants (crospovidone, sodium starch glycolate) was investigated with the aim to shorten the disintegration time of high-loaded sevelamer carbonate tablets. It was found with surprise that disintegrants did not shorten the disintegration time substantially. Sometimes, formulations with disintegrants showed actually slightly shorter disintegration time than comparable formulations without disintegrants, but, on the other hand, the hardness of the so produced tablets decreased to undesired levels due to compaction inhibiting properties of the disintegrants.

In case of starch, a filler known for swelling and tablet disintegrating properties, it was found that the disintegration time actually increased.

Therefore, the composition of the present invention preferably does not comprise any excipient that may serve as a disintegrant.
c) Use of a surfactant (sodium lauryl sulphate), which can improve the disintegration time sometimes, did not lead to shorter disintegration time either. Therefore, no surfactant is used in the composition of the invention.
d) Lastly, an excipient that may serve as a lubricant may be added into a composition. The lubricant improves tabletting properties of the composition and limits the adhesion of the composition to the tablet press. Suitable lubricant is, e.g., zinc stearate, magnesium stearate or stearic acid. The suitable amount of the lubricant is from 0.5 to 1.0 weight %.

The present invention also provides a process for making immediate release tablets comprising sevelamer carbonate, said process comprising tabletting, by a direct compression, a dry mixture of 70-85 weight per cent of sevelamer carbonate, calculated as an anhydrous compound, 10-25 weight percent of monohydrate of lactose and ,optionally, other excipients. The direct compression tabletting process comprises homogenization of the dry components in a suitable homogenizer and tabletting the homogeneous mixture in a tablet press under suitable tabletting pressure. The tabletting pressure may be adjusted to obtain preferably a tablet with a hardness of about 150 N and higher.

In particular, the process does not comprise any wetting step regardless the fact that wetting of sevelamer is sometimes recommended in the literature. Mostly, passive wetting by uptake of atmospheric moisture has been described to improve compactibility. This method is difficult to control and hardly applicable for commercial manufacturing with bulk quantities. Wetting of sevelamer by addition of water to a high shear mixer has also been described. This process requires considerable storage time for water equilibration after spraying and an additional milling step to get rid of lumps. Also it was found experimentally that sevelamer carbonate tends to form elastic lumps upon contact with water that could only be milled with conventional milling equipment upon hardening of the lumps after considerable storage time. Therefore, a direct compression process that does not require a wetting step is preferred.

The sevelamer carbonate tablets of the present invention are preferably film-coated to improve the handling properties and, particularly, to minimize the water uptake into the tablet during storage. Inherently, sevelamer carbonate tablet cores swell if the uptake of moisture occurs. Hence, a flexible protective coating is desired for high-loaded sevelamer carbonate tablets. Flexibility of the coating is particularly important for 'in-use' stability as patients might not always store the tablets according to the label recommendations.

In the second condition, the film coat should not increase the overall disintegration time in purified water . The film coat material should therefore be well soluble in water.

Three comparably soluble flexible coatings were evaluated experimentally for use with sevelamer carbonate tablet cores. Surprisingly it was found that use of polyvinyl alcohol-polyethylene glycol graft copolymer results in the lowest disintegration time of the overall composition. No explanation has been found for this observation.

The coating comprising polyvinyl alcohol-polyethylene glycol graft copolymer is commercially available under trade name Kollicoat IR by BASF. Apart of polyvinyl alcohol-polyethylene glycol graft copolymer, the coating material may comprise also other excipients improving the appearance and processability, such as colourants (e.g. titanium dioxide), anti-tacking agents (e.g. talc, kaolin) and glidants (e.g. silicon dioxide).

The advantage of the Kollicoat -type coatings over the most commonly used coatings based on polyvinylalcohol (PVA) and hypromellose (HPMC) was demonstrated by comparing the gains of the disintegration time (measured according to Ph.Eur. 2.9.1. in purified water without using the disk) after coating the tablet core prepared according to the invention with 3 % of the respective coatings (see Example C). While the PVA- and HPMC- type coating increases the disintegration time by about 5-10 minutes, the same amount of the Kollicoat coating increases the disintegration time by about 2 minutes only.

Accordingly, suitable film-coated high-loaded tablets comprising sevelamer carbonate and lactose monohydrate advantageously comprise 2-10 % , preferably about 2.5-5 % and most preferably about 3 % of the coating comprising polyvinyl alcohol-polyethylene glycol graft copolymer.

The tablet cores may be coated by a standard coating process by spraying a water suspension of the coating material at the set temperature in a coating equipment, e.g. in a conventional drum coater, until the desired weight gain is obtained. Kollicoat IR can be used for coating in a broader temperature range (15-50°C) than HPMC and PVA coatings, particularly at lower temperatures. Coating at low temperatures saves energy and improves the uniformity of the coating. Kollicoat IR has relatively low viscosity and can therefore be used for coating fluids with high solid content (15-25% polymer concentration and 20-35% overall solids with pigments). The high solid content reduces processing time in the manufacture of film-coated tablets. Accordingly, it can be concluded that the use of polyvinyl alcohol-polyethylene glycol graft copolymer, e.g. Kollicoat IR, for making coated tablets comprising sevelamer, particularly cevelamer carbonate, which use is one of the aspects of the present invention, is advantageous not only from the point of coating properties but also from technological aspects.

The tablets of the present invention are useful as a medicament for the control and/or adjustment of the level of serum phosphorus, particularly in patients with Chronic Kidney Disease (CKD). The phosphate binding capacity is comparable with that of the pure active ingredient and with the currently marketed sevelamer carbonate drug product (Renvela).

The results of experiments showing the superiority of the compositions and tablets of the present invention are summarized in the following Examples .

### EXAMPLES

### Example A (not according to the invention): Comparison of tablet core formulations

Tablets core formulations were compared with regard to disintegration time. To enable scientifically sound comparison of the various formulations, blends were prepared according to the same procedure and compacting was done with the same equipment, equipment settings, tooling and compression force.

Tablets were prepared according to the following procedure:
Sevelamer and colloidal silicon dioxide were blended (Turbula blender, 5 min) and screened. Remaining excipients - except lubricant - were added and blended (15 minutes). Lubricant was added for final blending (5 min). Blend was compressed with 30 kN compression force using excenterpress (Korsch EK-0) equipped with 10x19 mm oblong shaped tooling.

Disintegration time of tablets was determined by using a conventional tablet disintegration apparatus (Pharmatest) in accordance with the European Pharmacopoeia (Ph.Eur 2.9.1) in purified water without using the disk.

**Table A Formulations based on MCC, starch and disintegrants (amounts in milligram per tablet)**

| | A | B | D | E | F | G | K |
|---|---|---|---|---|---|---|---|
| Sevelamer carbonate | 800 | 800 | 800 | 800 | 800 | 800 | 800 |
| microcrystalline cellulose (Avicel PH105) | | 105 | 105 | | 105 | 105 | |
| silicified MCC (Prosolv HD90) | | 134.5 | 103.0 | 239.5 | 103 | 61 | |
| starch (Starch 1500) | | | | | | | 239.5 |
| crospovidon (Polyplasdone XL) | | | 31.5 | | | | |
| sodium starch glycolate (Primojel) | | | | | 31.5 | 73.5 | |
| colloidal silicon dioxide (Aerosil 200VV) | 4 | 5.25 | 5.25 | 5.25 | 5.25 | 5.25 | 5.25 |
| zinc stearate | 4 | 5.25 | 5.25 | 5.25 | 5.25 | 5.25 | 5.25 |
| *Total weight (mg):* | *808* | *1050* | *1050* | *1050* | *1050* | *1050* | *1050* |
| | | | | | | | |
| Hardness (N) | 145 | 296 | 285 | 291 | 292 | 266 | 196 |
| Disintegration time (min) | < 24 | < 10 | <9 | < 12 | < 10 | < 13 | > 30 |

**Table B Formulations based on various soluble binders (1) (amounts in milligram per tablet)**

| | M | Q | R | T | V | W |
|---|---|---|---|---|---|---|
| Sevelamer carbonate | 800 | 836* | 836* | 836* | 836* | 836* |
| co-povidone (Kollidon VA64) | 239.5 | | | 30 | | |
| mannitol (Pearlitol 200SD) | | 151.5 | 151.5 | 151.5 | 250.25 | 250.25 |
| povidone (Kollidon K30) | | | 30 | | | |
| colloidal silicon dioxide (Aerosil 200VV) | 5.25 | 5 | 5 | 5 | 5.5 | 5.5 |
| Magnesium stearate | | | | | | 8.25 |
| Zinc stearate | 5.25 | 7.5 | 7.5 | 7.5 | 8.25 | |
| *Total weight (mg):* | *1050* | *1000* | *1030* | *1030* | *1100* | *1100* |
| | | | | | | |
| Hardness (N) | 244 | 143 | 128 | 177 | 188 | 180 |
| Disintegration time (min) | > 30 | <9 | > 30 | < 25 | < 8 | <7 |

| | | | | | | |
|---|---|---|---|---|---|---|
| * 800 mg anhydrous Sevelamer after water content correction of 4.5% | | | | | | |

**Table C Formulations based on various soluble binders (2) (amounts in milligram per tablet)**

| | X | CC | BB | Z | AA |
|---|---|---|---|---|---|
| Sevelamer carbonate | 836* | 836* | 836* | 836* | 836* |
| compressible sucrose (Compressuc MS) | 250.25 | 250.25 | | | |
| isomalt (GalenIQ 721) | | | 250.25 | | |
| isomalt (GalenIQ 720) | | | | 250.25 | 250.25 |
| crospovidone (Kollidon CL) | | 33 | | | 33 |
| colloidal silicon dioxide (Aerosil 200VV) | 5.5 | 5.5 | 5.5 | 5.5 | 5.5 |
| Magnesium stearate | 8.25 | 8.25 | 8.25 | 8.25 | 8.25 |
| *Total weight (mg):* | *1100* | *1133* | *1100* | *1100* | *1133* |
| | | | | | |
| Hardness (N) | 190 | 134 | 153 | 191 | 169 |
| Disintegration time (min) | < 20 | < 11 | < 16 | < 16 | < 12 |

| | | | | | |
|---|---|---|---|---|---|
| * 800 mg anhydrous Sevelamer after water content correction of 4.5% | | | | | |

**Table D Formulations based on lactose (amounts in milligram per tablet)**

| | GG | HH | II | KK | BBB | MM | NN |
|---|---|---|---|---|---|---|---|
| Sevelamer carbonate | 836* | 836* | 836* | 836* | 836* | 836* | 836* |
| anhydrous lactose (Supertab 22AN) | 250.25 | | | | | | |
| lactose monohydrate (Supertab 11SD; spray dried) | | 250.25 | 250.25 | | | | |
| lactose monohydrate (Supertab 30GR; granulated) | | | | 250.25 | 52.75 | 217.25 | 150.25 |
| crospovidon (Kollidon CL) | | | 33 | | | | |
| povidone low viscosity (PVP K12) | | | | | | 33 | |
| HPC low viscosity (Klucel EF) | | | | | | | 100 |
| colloidal silicon dioxide (Aerosil 200VV) | 5.5 | 5.5 | 5.5 | 5.5 | 4.5 | 5.5 | 5.5 |
| Magnesium stearate | 8.25 | 8.25 | 8.25 | 8.25 | | | |
| Zinc stearate | | | | | 6.75 | 8.25 | 8.25 |
| *Total weight (mg):* | *1100* | *1100* | *1133* | *1100* | *900* | *1100* | *1100* |
| | | | | | | | |
| Hardness (N) | 175 | 182 | 162 | 141 | 166 | 157 | 143 |
| Disintegration time (min) | < 16 | < 8 | < 6 | < 4 | < 11 | < 14 | < 19 |

| | | | | | | | |
|---|---|---|---|---|---|---|---|
| * 800 mg anhydrous Sevelamer after water content correction of 4.5% | | | | | | | |

**Table E Formulation with surfactant (amounts in milligram per tablet)**

| | RR | KK |
|---|---|---|
| Sevelamer carbonate | 836* | 836* |
| lactose monohydrate (Supertab 30GR) | 239.25 | 250.25 |
| sodium lauryl sulfate (Texapon K12) | 11 | |
| colloidal silicon dioxide (Aerosil 200VV) | 5.5 | 5.5 |
| Zinc stearate | 8.25 | 8.25 |
| *Total weight (mg):* | *1100* | *1100* |
| | | |
| Hardness (N) | 141 | 141 |
| Disintegration time (min) | <6 | <4 |

| | | |
|---|---|---|
| * 800 mg anhydrous Sevelamer after water content correction of 4.5% | | |

### Example B (not according to the invention): Stability of phosphate binding capacity and disintegration time of lactose formulation

The phosphate binding capacity and disintegration time of Sevelamer tablets with lactose as binder were found to be stable. Stability studies were performed in accelerated conditions (40°C/75%RH) and stress conditions (55°/90%).

**Tablets of stability studies (amounts in milligram per tablet)**

| | AAA |
|---|---|
| Sevelamer carbonate | 836* |
| lactose monohydrate (Supertab 11SD) | 239.25 |
| colloidal silicon dioxide (Aerosil 200VV) | 5.5 |
| zinc stearate | 8.25 |
| *Total weight (mg):* | *1100* |

| | |
|---|---|
| * 800 mg anhydrous Sevelamer after water content correction of 4.5% | |

The tablets were packed in regular HPDE containers. The phosphate binding remained unchanged and the disintegration time decreased slightly.

### Stability of disintegration time (minutes)

| **Storage condition** | **t = 0** | **t = 1 month** |
|---|---|---|
| 40°/75% | < 11 min | < 8 min |
| 55°/90% | < 11 min | < 7 min |

### Stability of phosphate binding capacity (mmol PO₄/g SVL)

| **Storage condition** | **t = 0** | **t = 1 month** |
|---|---|---|
| 40°/75% | 5.7 | 5.8 |
| 55°/90% | 5.7 | 5.8 |

### Example C (not according to the invention): Coating of lactose-based tablets with PVA-based coating, with Kollicoat IR based coating, and with HPMC based coating

Tablets were prepared on equipment suitable for industrial manufacturing according to the following procedure:
Sevelamer carbonate and colloidal silicon dioxide were blended (Bohle free-fall blender, 5 min) and screened. Remaining excipients - except lubricant - were added and blended (15 minutes). Lubricant was added for final blending (5 min). Blend was compressed with rotary press (Korsch PH106) equipped with 11x18.5 mm oblong shaped tooling.

The tablet cores were coated to a weight gain of 3% in a conventional drum coater (Bohle BLC5) at a product temperature of ± 45°C for the PVA-based coating, at ± 30°C and ± 42°C for the Kollicoat IR based coating and at ±42°C for the HPMC based coating.

Disintegration time of tablets was determined by using a conventional tablet disintegration apparatus in accordance with the European Pharmacopoeia (Ph.Eur 2.9.1) in purified water without using the disk.

**Tablet formulations of comparative coating trials (amounts in milligram per tablet)**

| PVA-based coating | BBB1 | Kollicoat IR based coating | DDD | HPMC based coating | FFF |
|---|---|---|---|---|---|
| Sevelamer carbonate | 836* | Sevelamer carbonate | 836* | Sevelamer carbonate | 836* |
| lactose monohydrate (Supertab 30GR) | 250.2 5 | lactose monohydrate (Supertab 30GR) | 250.25 | lactose monohydrate (Supertab 30GR) | 250.25 |
| colloidal silicon dioxide (Aerosil 200VV) | 5.5 | colloidal silicon dioxide (Aerosil 200VV) | 5.5 | colloidal silicon dioxide (Aerosil 200VV) | 5.5 |
| zinc stearate | 8.25 | zinc stearate | 8.25 | zinc stearate | 8.25 |
| *Core weight (mg):* | *1100* | *Core weight (mg):* | *1100* | *Core weight (mg):* | *1100* |
| Opadry PVA 85F18422¹ | 33 | Kollicoat IR | 19.8 | HPMC low viscosity type 2910 | 12.71 |
| | | Titanium dioxide | 8.25 | HPMC high viscosity type 2910 | 12.71 |
| | | Talc | 4.95 | Diacetylated monoglycerides | 7.59 |
| *Total weight (mg):* | *1133* | *Total weight (mg):* | *1133* | *Total weight (mg):* | *1133* |

| | | | | | |
|---|---|---|---|---|---|
| * 800 mg anhydrous Sevelamer after water content correction of 4.5% ¹ containing: polyvinyl alcohol, titanium dioxide, polyethyleneglycol, talc | | | | | |

A substantial increase of disintegration time was seen with PVA-based coating and with HPMC-based coating. A smaller increase of disintegration time was seen with Kollicoat IR based coating:

### Disintegration time of lactose-based tablets before and after coating

| | **Opadry PVA** | **Kollicoat IR 30°C** | **Kollicoat IR 42°C** | **HPMC** |
|---|---|---|---|---|
| Before coating | < 15 min | < 11 min | < 11 min | < 13 min |
| After coating | < 22 min | < 13 min | < 13 min | < 21 min |

### Example 1 - Tablet cores:

Composition (amounts in miligrams per tablet) :

| | |
|---|---|
| Sevelamer carbonate | 836* |
| lactose monohydrate (Supertab 30GR) | 250.2 5 |
| colloidal silicon dioxide (Aerosil 200VV) | 5.5 |
| zinc stearate | 8.25 |
| *Core weight (mg):* | *1100* |

| | |
|---|---|
| * corresponds to 800 mg anhydrous Sevelamer carbonate after water content correction of 4.5% | |

### Process:

Sevelamer carbonate and colloidal silicon dioxide were blended (Turbula blender, 5 min) and screened. Lactose monohydrate was added and blended (15 minutes). Lubricant was added for final blending (5 min). Blend was compressed with 30 kN compression force using excenterpress (Korsch EK-0) equipped with 10x19 mm oblong shaped tooling.

Disintegration time according to the Ph.Eur. 2.9.1. in purified water without using the disk: less than 10 minutes

### Example 2 - Coated tablets

Composition (amounts in miligrams per tablet) :

| | |
|---|---|
| Sevelamer carbonate | 836* |
| lactose monohydrate (Supertab 30GR) | 250.2 5 |
| colloidal silicon dioxide (Aerosil 200VV) | 5.5 |
| zinc stearate | 8.25 |
| *Core weight (mg):* | *1100* |
| Kollicoat IR | 19.8 |
| Titanium dioxide | 8.25 |
| Talc | 4.95 |
| *Total weight (mg):* | *1133* |

| | |
|---|---|
| * corresponds to 800 mg anhydrous Sevelamer carbonate after water content correction of 4.5% | |

### Process :

The tablet cores prepared according to the formulation of the example 1 were coated to a weight gain of 3% in a conventional drum coater (Bohle BLC5) at a product temperature of ± 30°C by the Kollicoat IR based coating.

The invention having been described it will be obvious that the same may be varied in many ways and all such modifications are contemplated as being within the scope of the invention as defined by the following claims.

## Claims

1. A pharmaceutical immediate release tablet comprising a core comprising 70-85 weight per cent of sevelamer carbonate, calculated as an anhydrous compound, 10-25 weight percent of lactose monohydrate and, optionally, a water soluble film coat surrounding the core, wherein the composition is free of crystalline cellulose, and (low-substituted) hydroxypropyl cellulose or other binding agents.

2. The tablet according to claim 1, which comprises 800 mg of sevelamer carbonate.

3. The tablet according to claim 1 or 2, wherein the total mass of the tablet, including the optional coating, does not exceed 1150 mg.

4. The tablet according to any one of claims 1 to 3, wherein the tablet is coated and the film coat comprises polyvinyl alcohol-polyethylene glycol graft copolymer.

5. The tablet according to any one of claims 1 to 4, wherein the amount of the film coat is 2-10% of the weight of the tablet core.

6. The tablet according to any one of claims 1 to 5, not comprising an added monovalent anion source and/or a disintegrant and/or a surfactant.

7. The tablet according to any one of claims 1 to 6, having a hardness of about 150 N and higher.

8. The tablet according to any one of claims 1 to 7, having a disintegration of less than 10 minutes using a conventional tablet disintegration apparatus (Pharmatest) in accordance with the European Pharmacopoeia (Ph.Eur 2.9.1) in purified water without using the disk.

9. A process for making immediate release tablets comprising sevelamer carbonate, said process comprising tabletting, by a direct compression, a dry mixture of 70-85 weight per cent of sevelamer carbonate, calculated as an anhydrous compound, 10-25 weight percent lactose monohydrate and ,optionally, other excipients, wherein the composition is free of crystalline cellulose, and (low-substituted) hydroxypropyl cellulose or other binding agents.

10. The process according to claim 9, wherein the tabletting step is followed by a step of coating the formed tablets by a water soluble film coat.

11. The process according to claim 9 or 10, wherein the water soluble film coat comprises polyvinyl alcohol-polyethylene glycol graft copolymer.

12. Tablets according to any one of claims 1 to 8 and/or prepared according to the process of any one of claims 9 to 11 for use as medicament for the control and/or adjustment of the level of serum phosphorus in patients with Chronic Kidney Disease.

## Patentansprüche

1. Pharmazeutische Tablette mit sofortiger Freisetzung, umfassend einen Kern, umfassend 70-85 Gewichtsprozent Sevelamercarbonat, berechnet als wasserfreie Verbindung, 10-25 Gewichtsprozent Lactosemonohydrat und, optional, eine den Kern umgebende wasserlösliche Filmbeschichtung, wobei die Zusammensetzung frei von kristalliner Cellulose und (niedrig substituierter) Hydroxypropylcellulose oder anderen Bindemitteln ist.

2. Tablette nach Anspruch 1, die 800 mg Sevelamercarbonat enthält.

3. Tablette nach Anspruch 1 oder 2, wobei die Gesamtmasse der Tablette, einschließlich der optionalen Beschichtung, 1150 mg nicht überschreitet.

4. Tablette nach einem der Ansprüche 1 bis 3, wobei die Tablette beschichtet ist und die Filmbeschichtung ein Polyvinylalkohol-Polyethylenglykol-Pfropfcopolymer enthält.

5. Tablette nach einem der Ansprüche 1 bis 4, wobei die Menge der Filmbeschichtung 2-10 % des Gewichts des Tablettenkerns beträgt.

6. Tablette nach einem der Ansprüche 1 bis 5, die keine zugesetzte monovalente Anionenquelle und/oder Sprengmittel und/oder Tensid enthält.

7. Tablette nach einem der Ansprüche 1 bis 6 mit einer Härte von etwa 150 N und mehr.

8. Tablette nach einem der Ansprüche 1 bis 7, die einen Zerfall von weniger als 10 Minuten unter Verwendung einer herkömmlichen Tablettenauflösungsvorrichtung (Pharmatest) gemäß dem Europäischen Arzneibuch (Ph.Eur 2.9.1) in gereinigtem Wasser ohne Verwendung der Scheibe aufweist.

9. Verfahren zur Herstellung von Tabletten mit sofortiger Freisetzung, die Sevelamercarbonat enthalten, wobei das Verfahren Tablettieren, durch direktes Komprimieren, einer trockenen Mischung aus 70-85 Gewichtsprozent Sevelamercarbonat, berechnet als wasserfreie Verbindung, 10-25 Gewichtsprozent Lactosemonohydrat und gegebenenfalls anderer Hilfsstoffe umfasst, wobei die Zusammensetzung frei von kristalliner Cellulose und (niedrig substituierter) Hydroxypropylcellulose oder anderen Bindemitteln ist.

10. Verfahren nach Anspruch 9, wobei auf den Tablettierungsschritt ein Schritt des Beschichtens der gebildeten Tabletten durch eine wasserlösliche Filmbeschichtung folgt.

11. Verfahren nach Anspruch 9 oder 10, wobei die wasserlösliche Filmbeschichtung ein Polyvinylalkohol-Polyethylenglykol-Pfropfcopolymer enthält.

12. Tabletten nach einem der Ansprüche 1 bis 8 und/oder hergestellt gemäß dem Verfahren nach einem der Ansprüche 9 bis 11 zur Verwendung als Medikament zur Kontrolle und/oder Einstellung des Serumphosphorspiegels bei Patienten mit chronischer Nierenerkrankung.

## Revendications

1. Comprimé pharmaceutique à libération immédiate comprenant un noyau comprenant 70-85 pour cent en poids de carbonate de sevelamer, calculé en tant que composé anhydre, 10-25 pour cent en poids de lactose monohydraté et, en option, un enrobage pelliculaire hydrosoluble entourant le noyau, dans lequel la composition ne contient pas de cellulose cristalline ni d'hydroxypropylcellulose (à faible degré de substitution) ni d'autres agents liants.

2. Comprimé selon la revendication 1, qui comprend 800 mg de carbonate de sevelamer.

3. Comprimé selon la revendication 1 ou 2, dans lequel la masse totale du comprimé, y compris l'enrobage facultatif, ne dépasse pas 1150 mg.

4. Comprimé selon l'une quelconque des revendications 1 à 3, dans lequel le comprimé est enrobé et l'enrobage pelliculaire comprend un copolymère greffé d'alcool polyvinylique-polyéthylène glycol.

5. Comprimé selon l'une quelconque des revendications 1 à 4, dans lequel la quantité d'enrobage pelliculaire représente 2-10% du poids du noyau du comprimé.

6. Comprimé selon l'une quelconque des revendications 1 à 5, ne comprenant pas une source d'anions monovalente ajoutée et/ou un désintégrateur et/ou un surfactant.

7. Comprimé selon l'une quelconque des revendications 1 à 6, ayant une dureté d'environ 150 N et plus.

8. Comprimé selon l'une quelconque des revendications 1 à 7, présentant une désintégration de moins de 10 minutes à l'aide d'un appareil de désintégration de comprimé conventionnel (Pharmatest) conforme à la Pharmacopée Européenne (Ph.Eur 2.9.1) dans de l'eau purifiée sans utiliser le disque.

9. Procédé de fabrication de comprimés à libération immédiate comprenant du carbonate de sevelamer, ledit procédé comprenant la compression, par une compression directe, d'un mélange sec de 70-85 pour cent en poids de carbonate de sevelamer, calculé en tant que composé anhydre, 10-25 pour cent en poids de lactose monohydraté et, en option, d'autres excdipients, dans lequel la composition ne contient pas de cellulose cristalline ni d'hydroxypropylcellulose (à faible degré de substitution) ni d'autres agents liants.

10. Procédé selon la revendication 9, dans lequel l'étape de compression est suivie d'une étape d'enrobage des comprimés formée par une pellicule soluble dans l'eau.

11. Procédé selon la revendication 9 ou 10, dans lequel l'enrobage pelliculaire soluble dans l'eau comprend un copolymère greffé d'alcool polyvinylique polyéthylène glycol.

12. Comprimés selon l'une quelconque des revendications 1 à 8 et/ou préparés selon le procédé de l'une quelconque des revendications 9 à 11, destinés à être utilisés en tant que médicament pour le contrôle et/ou l'ajustement du taux de phosphore sérique chez les patients atteints de Maladie Rénale Chronique.
